# EUROPÄISCHE PATENTSCHRIFT

(11) **EP 3 482 187 B1**
(45) Veröffentlichungstag und Bekanntmachung des Hinweises auf die Patenterteilung: **05.05.2021**
(21) Anmeldenummer: 17758039.6
(22) Anmeldetag: 05.07.2017
(51) Int. Cl.: G01N 1/22, B08B 9/46, G01N 21/90, B07C 5/34

(54) **VORRICHTUNG UND VERFAHREN ZUM UNTERSUCHEN VON BEHÄLTERN AUF FREMDSTOFFE**
DEVICE AND METHOD FOR INSPECTING CONTAINERS FOR THE PRESENCE OF FOREIGN MATTER
DISPOSITIF ET PROCÉDÉ POUR ANALYSER DES RÉCIPIENTS QUANT À LA PRÉSENCE DE CORPS ÉTRANGERS

(30) Priorität: 06.07.2016 DE 102016212321
(43) Veröffentlichungstag der Anmeldung: 15.05.2019
(73) Patentinhaber: Krieg, Gunther, Prof.Dr.Ing., 76227 Karlsruhe (DE)
(72) Erfinder: BOHLEBER, Juergen, 77815 Bühl (DE); FEY, Dirk, 67630 Neewiller (FR); FRODL, Tobias, 76316 Malsch (DE); KRIEG, Gunther, 76227 Karlsruhe (DE)
(74) Vertreter: Patent- und Rechtsanwälte Ullrich & Naumann
(86) Internationale Anmeldenummer: PCT/DE2017/200062
(87) Internationale Veröffentlichungsnummer: WO 2018/006910

(56) Entgegenhaltungen:
- EP-A1- 1 619 493
- DE-A1-102004 048 146
- GB-A- 2 006 430
- US-A- 6 013 228

## Beschreibung

Die vorliegende Erfindung betrifft eine Vorrichtung zum Untersuchen von Behältern auf Fremdstoffe, gemäß Anspruch 1.

Die Erfindung betrifft ferner ein Verfahren zum Untersuchen von Behältern auf Fremdstoffe, gemäß Anspruch 10.

Obwohl auf beliebigen Gebieten anwendbar, wird die vorliegende Erfindung in Bezug auf Mehrfach-Verwendung von Behältern erläutert.

Obwohl auf beliebige Behälter anwendbar, wird die vorliegende Erfindung in Bezug auf Flaschen, beispielsweise Mehrweg-Flaschen erläutert.

Obwohl auf beliebige Fremdstoffe anwendbar, wir die vorliegende Erfindung mit Bezug auf Benzin erläutert.

Der Einsatz von Kunststoffen im Mehrweg-Bereich erfährt in letzter Zeit eine wachsende Bedeutung, insbesondere lateinamerikanischen Ländern, wie Mexico, etc. Kunststoffmüll hat bereits eine erhebliche Auswirkung auf die Umwelt, sei es durch die Verschmutzung von Gewässern oder die immer weiter ansteigende Menge an Kunststoffmüll. Trotzdem erfreuen sich im Bereich der Getränkeindustrie Kunststoffflaschen nach wie vor großer Beliebtheit, nicht nur wegen ihres in Bezug auf Glasflaschen deutlich geringeren Gewichts, sondern auch wegen ihrer Robustheit. Mehrwegflaschen, also Flaschen, die im Laufe Ihres Lebens mehrfach mit Getränken (wieder) befüllt und verkauft werden, werden dabei von einigen Endverbrauchern auch zweckentfremdet benutzt. So werden Mehrwegkunststoffflaschen auch benutzt, um darin Öl, Benzin, Farben oder Ähnliches zwischenzeitlich aufzubewahren. Durch Ihre bedingte Dehnungsfähigkeit bei überhöhtem Druck weisen diese im Vergleich zu Glasflaschen auch eine geringere Gefährlichkeit hinsichtlich Explosion, etc. auf. Nichtsdestotrotz finden sich derart benutzte Flaschen auch im Recyclingkreislauf wieder. Da diese Flaschen jedoch die früher enthaltenen Fremdstoffe selbst nach entsprechendem Reinigen der Flasche teilweise zurückbehalten können, beispielsweise da gewisse Stoffe lipophil sind und in den Kunststoff diffundieren, dürfen diese Flaschen nicht mehr für Getränke bzw. Lebensmittel weiterverwendet werden - diese Stoffe können bei einem Wiederbefüllen bspw. einen Fehlgeschmack verursachen - und müssen zuverlässig aussortiert werden.

Aus der DE 10 2004 048 146 A1 ist eine Vorrichtung zum Untersuchen von Behältern auf Fremdstoffe bekannt geworden. Hierbei wird über einen Probenahmekopf und über eine Druckluftlanze nach Aufsetzen des Probenahmekopfes auf eine Flasche Luft eingeblasen. Über eine Entnahmeleitung kann dann Gas aus der Flasche entnommen und das entnommene Gas einem Analysesystem zur weiteren Untersuchung zugeführt werden.

Aus der US 6,013,228 A sind ein Verfahren und eine Vorrichtung zum Untersuchen von Rückständen in Behältern bekannt. Dabei wird der Behälter mit Druckluft beaufschlagt und eine Probe der aus dem Behälter dadurch ausströmenden Luft analysiert, wobei die Probe ohne aufsetzten des Probenahmekopfes entnommen wird. Die Vorrichtung enthält ein Filter und ein Drucksensor.

Problematisch hierbei ist, dass Flaschen nicht nur mit Fremdstoffen verunreinigt sein können, sondern auch physische Beschädigungen in Form von Löchern, Abbruchkanten oder dergleichen aufweisen, insbesondere im Bereich der Flaschenöffnung. Wird nun der Probenahmekopf aufgesetzt und Druckluft eingeblasen, kann über derartige Beschädigungen beispielsweise Fremdluft in die Flasche gelangen und so das Messergebnis an dem entnommenen Gas verfälschen. Darüber hinaus kann auch aus der Fehlstelle das entnommene Gas ganz oder zumindest teilweise entweichen, also dann nicht oder nicht vollständig zum Analysesystem gelangen, was das Messergebnis verfälschen kann. Kontaminierte Flaschen werden so nicht aussortiert und gelangen wieder in die Abfüllung zur erneuten Befüllung mit Getränken.

Eine Aufgabe der vorliegenden Erfindung ist es daher, die Zuverlässigkeit bei der Erkennung von Fremdstoffen in Behältern zu erhöhen ohne den Herstellungsaufwand und die Kosten wesentlich zu vergrößern.

Die vorliegende Erfindung löst die vorstehend genannte Aufgabe bei einer Vorrichtung zum Untersuchen von Behältern auf Fremdstoffe, gemäß Anspruch 1.

Die vorliegende Erfindung löst die vorstehende Aufgabe ebenfalls bei einem Verfahren zum Untersuchen von Behältern auf Fremdstoffe, gemäß Anspruch 10. des Drucks, bei der Entnahme aus dem Behälter gemessen wird.

Unter dem Begriff "Fremdstoff" ist jeglicher Stoff oder jegliches Stoffgemisch zu verstehen, der bzw. das nicht zweckgemäß in Bezug auf den Behälter gebraucht oder eingesetzt wird. Beispielsweise ist bei einer Getränkekunststoffmehrwegflasche der bestimmungs- oder zweckgemäße Gebauch der Flasche auf die Aufbewahrung von für Menschen ohne wesentliche Gesundheitsgefahren trinkbare Stoffe begrenzt. Fremdstoffe für Getränkekunststoffmehrwegflaschen sind beispielsweise Benzin, Öl, Urin, Lacke, Lösungsmittel, etc..

Unter dem Begriff "Fluid" ist jeglicher Stoff oder jegliches Stoffgemisch zu verstehen, welches sich in gasförmigem und/oder flüssigem Aggregatzustand befindet.

Unter dem Begriff "zeitlicher Verlauf des Drucks" ist jegliche Änderung des Drucks, insbesondere ein Anstieg und/oder ein Absinken des Drucks über eine vorgebbare Zeit zu verstehen. Der zeitliche Druckverlauf kann hier in Form einer Gerade, Kurve, etc. vorliegen. Weiterhin kann die Auswertung des Drucks bzw. Druckverlaufs auf die ansteigende und/oder die fallende Flanke des Druckverlaufs beschränkt sein.

Unter dem Begriff "Druckmessung" ist jegliche Messung von einem oder mehreren Drücken zu verstehen. Insbesondere ist hierunter auch eine Messung eines Minimal- und/oder Maximaldrucks eines zeitlichen Verlaufs eines Drucks zu verstehen.

Einer der mit der Erfindung erzielten Vorteile ist, dass mittels des Drucksensors eine Überwachung des Drucks des entnommenen zweiten Fluids möglich ist, so dass beispielsweise, wenn gleichzeitig das erste Fluid eingebracht und das zweite Fluid entnommen wird, unregelmäßige Veränderungen des Drucks des zweiten Fluids insbesondere anhand des zeitlichen Verlaufs des Drucks des zweiten Fluids, die beispielsweise auf ein Loch, abgebrochene Kanten, ein ungleichmäßiges Aufsetzen des Probenahmekopfes, etc., zurückzuführen sind, zuverlässig detektiert werden können. Damit wird insgesamt die Zuverlässigkeit der Erkennung von Fremdstoffen in Behältern wesentlich erhöht. Darüber hinaus sind damit auch keine wesentlichen zusätzlichen Kosten verbunden, denn eine Implementierung zumindest eines Drucksensors ist auf einfache und kostengünstige Weise möglich.

Weitere Merkmale, Vorteile und bevorzugte Ausführungsformen der Erfindung sind in den folgenden Unteransprüchen beschrieben oder werden durch diese offenbar.

Erfindungsgemäß ist eine Analyseeinrichtung zur Analyse des mittels des zumindest einen Drucksensors gemessenen Drucks des zweiten Fluids und zur Analyse des zweiten Fluids in Bezug auf Fremdstoffe angeordnet. Damit ist eine kostengünstige Realisierung der Druckanalyse des zweiten Fluids möglich. Eine separate zweite Analyseeinrichtung wird nicht benötigt.

Zweckmäßigerweise ist der zumindest eine Drucksensor fluidtechnisch stromabwärts des Probenahmekopfes und stromaufwärts der Analyseeinrichtung angeordnet. Durch die Anordnung zwischen Probenahmekopf und Analyseeinrichtung wird eine besonders genaue Messung, insbesondere nah an dem Probenahmekopf ermöglicht. Verfälschungen durch Druckabfall, etc. innerhalb der Analyseeinrichtung werden so vermieden.

Vorteilhafterweise ist die Analyseeinrichtung ausgebildet, mittels einer oder mehrerer spektroskopischer Analysen des zweiten Fluids Fremdstoffe zu ermitteln. Eine spektroskopische Analyse des zweiten Fluids ermöglicht eine schnelle und gleichzeitig äußerst zuverlässige Bestimmung von Fremdstoffen in den Behältern.

Zweckmäßigerweise ist eine Fördereinrichtung für die Behälter angeordnet, derart, dass die Bewegung des Probenahmekopfes und die Bewegung der Behälter miteinander synchronisierbar sind. Dabei kann beispielsweise die Bewegung von Probenahmekopf und Behälter elektronisch oder auch mechanisch synchronisiert werden. Hier kann beispielsweise bei einem separaten Antrieb für die Mitbewegung des Probenahmekopfes in bevorzugter Ausgestaltung eine elektronische Synchronisationseinrichtung angeordnet werden. Alternativ können bei einer lediglich getrieblichen Verbindung des Probenahmekopfes zum Antrieb der Fördereinrichtung für die Behälter die Bewegung des Behälters und die Mitbewegung des Probenahmekopfes mechanisch, beispielsweise über ein Getriebe, synchronisiert werden. Damit kann auf einfache Weise eine zuverlässige Probenentnahme erfolgen.

Erfindungsgemäß weist der Probenahmekopf einen Dichtkörper auf zum Abdichten der Behälteröffnung während der Einbringung des ersten Fluids und/oder während der Entnahme des zweiten Fluids. Ein Dichtkörper, der beispielsweise aus einem weichen und/oder geschäumten Material gefertigt ist, ermöglicht ein fluiddichtes Anliegen des Probenahmekopfes. Dabei kann der Probenahmekopf bzw. ein Teil des Probenahmekopfs als Kugelinnenfläche aus einem verschleißfesten gehärteten Edelstahl ausgeführt werden, so dass der Probenahmekopf in einem weiten Winkelbereich fluiddicht am Behälter anliegen kann. Letztere Ausführungsform ermöglicht einen geringen Verschleiß des Dichtkörpers und damit eine hohe Störsicherheit.

Vorteilhafterweise weist der Dichtkörper eine konische Bohrung auf. Damit kann der Strom der Fluide, insbesondere des zweiten Fluids im Probenahmekopf verbessert werden. Wird das zweite Fluid im Bereich der Behälteröffnung im Probenahmekopf mittels Bohrungen geführt, kann es zu einem teilweisen Verschließen der Bohrungen kommen, wenn beispielsweise der Dichtkörper nicht vollständig plan auf der Behälteröffnung aufliegt. Durch die konische Ausbildung wird zum einen die Dichtfläche des Dichtkörpers gegenüber dem Behälter möglichst groß, sodass auch bei nicht ganz planem Aufsetzen trotzdem eine im Wesentlichen fluiddichte Verbindung ermöglichst wird. Gleichzeitig ist auch die Querschnittsfläche gegenüber dem Probennahmekopf möglichst groß, sodass eventuelle vorhandene Bohrungen auch bei nicht ganz planem Aufsetzen nicht verdeckt und so die Strömung des zweiten Fluids nicht beeinträchtigt wird.

Zweckmäßigerweise besteht der Dichtkörper zumindest teilweise aus Gummi. Damit wird eine einfache, kostengünstige und zuverlässige Abdichtung ermöglicht. Erfindungsgemäß ist ein Filter zur Filterung des zweiten Fluids angeordnet. Damit wird vermeiden, dass Grobschmutz aus der Umgebung, insbesondere aus dem Behälter, der durch das Einbringen des ersten Fluids durch das zweite Fluid mittransportiert wird, in das Meßsystem gelangt und dieses verschmutzt. Damit läßt sich die Zuverlässigkeit der Vorrichtung wesentlich steigern. Darüber hinaus kann ein weiterer Filter stromabwärts des genannten ersten Filters, bspw. in der Entnahmeleitung angeordnet sein, welche eine weitere Filterung des zweiten Fluids bereitstellt. Dieser zweite Filter kann so angeordnet sein, dass eine besonders einfache Wartung durch einfache Zugänglichkeit desselben ermöglicht wird.

Erfindungsgemäß ist der Filter im Probenahmekopf angeordnet. Damit wird noch im Probenahmekopf vermieden, dass Schmutz in stromabwärts des Probenahmekopfes angeordnete Bereiche, Leitungen, etc. gelangt, nicht nur in das Messsystem.

Vorteilhafterweise ist eine Fluidbereitstellungseinrichtung angeordnet, welche ausgebildet ist, das erste Fluid in Form eines Gases oder Gasgemisches bereitzustellen. Dabei kann auf gleichzeitig einfache und zuverlässige Weise ein Fluid bereitgestellt werden.

Zweckmäßigerweise ist mittels der Fluidbereitstellungseinrichtung das Gas in Form von, insbesondere ölfreier, Luft bereitstellbar. Damit kann auf besonders günstige Weise, beispielsweise in dem einfach die Umgebungsluft unter Druck in den Behälter eingebracht wird, bereitgestellt werden.

Vorteilhafterweise ist der Drucksensor als Hochgeschwindigkeitsdrucksensor ausgebildet. Damit kann die Vorrichtung einer Vielzahl von zu untersuchenden Behältern, wie dies beispielsweise beim Prüfen von Mehrzweckflaschen auf Reinheit vor der Wiederbefüllung erforderlich ist, verwendet werden, was die Flexibilität hinsichtlich des Einsatzes der Vorrichtung erhöht.

Zweckmäßigerweise beträgt die Ansprechzeit des Drucksensors weniger als 50 ms, insbesondere weniger als 25ms, vorzugsweise weniger als 10ms, besonders vorzugsweise weniger als 5ms, insbesondere zwischen 1 und 3ms. Damit ist eine schnelle Messung des Drucks, insbesondere des zeitlichen Verlaufs des Drucks möglich, sodass hohe Taktraten, also viele Untersuchungen pro zeit von Flaschen auf Fremdstoffe, wie sie beispielsweise beim Wiederbefüllen beim Recycling von Getränkeflaschen auftreten, möglich sind.

Vorteilhafterweise ist das erste Fluid mittels des Probenahmekopfes zentral in den zumindest einen Behälter einbringbar. Damit kann eine besonders große Menge des zweites Fluids in kürzerer Zeit auf zuverlässige Weise aus dem Behälter in den Probenahmekopf eingebracht und der späteren Druckmessung, insbesondere einer Druckverlaufsmessung, und Analyse zugeführt werden.

Zweckmäßigerweise ist eine Sortiereinrichtung angeordnet, die ausgebildet ist, anhand eines Resultats der Analyseeinrichtung Behälter abweichend von einem vorab definierten Resultat auszusortieren. Damit können auf zuverlässige Weise Behälter, bei denen kein Resultat oder ein von einem vorab definierten Resultat abweichendes Resultat vorliegt, aussortiert werden. Das vorab definierte Resultat kann beispielsweise durch eine Vielzahl von Behältern in unterschiedlichen Restmengen und unterschiedlichen Fremdstoffen kalibriert werden, so dass durch Vergleich des hinterlegten Resultats mit dem gemessenen Resultat eine Aussortierung des entsprechenden Behälters möglich wird.

Vorteilhafterweise ist die Sensoreinrichtung ausgebildet, die Behälter zumindest einmal erneut der Untersuchung auf Fremdstoffe zuzuführen. Damit kann beispielsweise dem Umstand Rechnung getragen werden, dass ein von dem vorab definierten Resultat abweichendes Resultat nicht notwendigerweise eine Aussortierung rechtfertigt. In diesem Fall wird der Behälter temporär aussortiert und später erneut auf Fremdstoffe untersucht. In bevorzugter Weise kann die Anzahl der erneuten Untersuchungen vorab festgelegt werden. Weicht das Resultat bei jeder oder einer vorab definierten Anzahl, Prozentsatz oder dergleichen der erneuten Untersuchungen weiterhin von dem vorab definierten Resultat ab, kann der Behälter endgültig aussortiert werden und beispielsweise entweder direkt aus dem Recycling von dem bestimmungsgemäßen Gebrauch des Behälters entfernt werden oder aber gegebenenfalls einer manuellen Kontrolle, etc. zugeführt werden.

Zweckmäßigerweise wird Luft als erstes Fluid bereitgestellt. Damit kann auf besonders einfache und kostengünstige Weise ein erstes Fluid bereitgestellt werden.

Vorteilhafterweise werden Behälter zumindest einmal erneut der Untersuchung auf Fremdstoffe zugeführt, wenn ein Resultat einer Analyseeinrichtung bei Analyse des zweiten Fluids von einem vorab definierten Resultat abweicht. Damit können auf zuverlässige Weise Behälter, bei denen keine oder ein von einem vorab definierten Resultat abweichendes Resultat aussortiert werden. Das vorab definierte Resultat kann beispielsweise durch eine Vielzahl von Behältern in unterschiedlichen Restmengen und unterschiedlichen Fremdstoffen kalibriert werden, so dass durch Vergleich des hinterlegten Resultats mit dem gemessenen Resultat eine Aussortierung des entsprechenden Behälters möglich wird.

Zweckmäßigerweise sind mehrere Behälter gleichzeitig untersuchbar und die jeweiligen Drücke der entnommenen zweiten Fluide sind gleichzeitig mess- und analysierbar. Dies ermöglicht eine äußerst schnelle und effiziente Untersuchung von Behältern. Der Begriff "gleichzeitig" in Bezug auf die Untersuchung auf Fremdstoffe ist insbesondere in der Beschreibung, vorzugsweise in den Ansprüchen, derart zu verstehen, dass die Zeitdauer für zwei oder mehrere Untersuchungen auf Fremdstoffe unterschiedlicher Behälter so schnell erfolgt, dass diese für die Zeitdauer für die Bereitstellung weiterer Behälter im Wesentlichen unerheblich ist. Dies bedeutet insbesondere, dass eine "gleichzeitige" Messung von Drücken auch eine sequentielle Messung einzelner Drücke nacheinander beinhaltet, wobei die summierte Zeitdauer der Messungen wesentlich kleiner ist als das Bereitstellen weiterer neuer Behälter zur Untersuchung auf Fremdstoffe.

Vorteilhafterweise wird bei der Entnahme des zweiten Fluids der Druckverlauf des zweiten Fluids während des Einbringens des ersten Fluids gemessen. Dies ermöglicht eine besonders genaue Analyse des Drucks und damit auch, ob bspw. der Probenahmekopf ordnungsgemäß auf einem Behälter aufgesetzt wurde.

Zweckmäßigerweise wird der Druck, insbesondere der Druckverlauf, des ersten Fluids beim Einbringen in den zumindest einen Behälter gemessen. Damit wird die Zuverlässigkeit noch weiter gesteigert: Wird auch der Druckverlauf des ersten Fluids gemessen, kann festgestellt werden, ob der für das Einbringen vorherbestimmte Druck zur Verfügung gestellt werden kann.

Vorteilhafterweise werden die Druckverläufe sowohl des ersten Fluids als auch des zweiten Fluids gemessen und ausgewertet. Damit kann auf besonders zuverlässige Weise bspw. ein korrektes Aufsetzen des Probenahmekopfes auf den Behälter, ebenso wie ein korrektes Einbringen des ersten Fluids und Entnehmen des zweiten Fluids festgestellt werden.

Zweckmäßigerweise wird das zweite Fluid optisch spektroskopisch untersucht und anhand der erhaltenen Daten der spektroskopischen Untersuchung wird/werden mittels der Analyseeinrichtung der/die Fremdstoff/e ermittelt. Damit lassen sich mittels der Analyseeinrichtung bspw. sowohl die Drucküberwachung als auch die Analyse auf Fremdstoffe durchführen.

Vorteilhafterweise wird der Druck des zweiten Fluids, insbesondere des Druckverlaufs des zweiten Fluids, mittels der Analyseeinrichtung anhand eines Soll-Ist-Vergleichs von Druckwerten, insbesondere von Druckverläufen, analysiert, wobei bei Unter- und/oder Überschreiten eines vorgegebenen Wertes die Untersuchung des Behälters als fehlerhaft klassifiziert werden kann. Damit kann auf einfache und gleichzeitig zuverlässige und schnelle Weise eine Analyse des Drucks des zweiten Fluids durchgeführt werden.

Zweckmäßigerweise wird der Soll-Wert des Soll-Ist-Vergleichs anhand einer Durchschnittsbildung einer Vielzahl von Druckmessungen festgelegt. Der Durchschnitt kann hierbei als gleitender Durchschnitt, etc. ausgestaltet sein, beispielsweise indem für die Durchschnittsbildung lediglich die letzten 10.000 Behälter, berücksichtigt werden.

Vorteilhafterweise werden verschiedene Druckverläufe und/oder Durchschnittswerte von Druckmessungen zu unterschiedlichen Zeitpunkten analysiert. Darüber hinaus ist es möglich, verschiedene Druckverläufe bzw. deren Durchschnittswerte zu speichern und diese ggf. unabhängig von einer aktuellen laufenden Messung durch die Analyseeinrichtung analysieren zu lassen. Zeigt sich bspw. durch Vergleich des Durchschnittsdruckwerts von 10.000 Behältern vor 4 Wochen und dem heutigen Durchschnittsdruckwerts einer signifikante Abweichung, die vorab relativ oder absolut festgelegt werden kann, so können daraus bspw. Rückschlüsse auf eine Verschmutzung der Entnahme- oder Zuführleitung, von Filtern, etc. gezogen werden. Dies ermöglicht dann eine zeitnahe und trotzdem flexible Wartung der Vorrichtung je nach Bedarf. Je nach Ergebnis der Analyse lässt sich auch die Wartung auf lediglich die Filter, Reinigung der Entnahmeleitung, etc. eingrenzen.

Weitere wichtige Merkmale und Vorteile der Erfindung ergeben sich aus den Unteransprüchen, aus den Zeichnungen, und aus dazugehöriger Figurenbeschreibung anhand der Zeichnungen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Bevorzugte Ausführungen und Ausführungsformen der Erfindung sind in den Zeichnungen dargestellt und werden in der nachfolgenden Beschreibung näher erläutert, wobei sich gleiche Bezugszeichen auf gleiche oder ähnliche oder funktional gleiche Bauteile oder Elemente beziehen.

In den Fig. zeigen
- Fig. 1: in schematischer Form eine Darstellung einer Vorrichtung die nicht Teil der Erfindung ist;

- Fig. 2: einen prinzipiellen Aufbau einer Vorrichtung gemäß einer Ausführungsform der vorliegenden Erfindung;
- Fig. 3: Ergebnisse einer Druckmessung 1,5 Liter REFPET-Flaschen bei einer Geschwindigkeit von 36.000 Flaschen pro Stunde während einer Zeitdauer von 30 Minuten;
- Fig. 4: in schematischer Form einen Ausschnitt einer Ausführungsform der vorliegenden Erfindung im Bereich des Probenahmekopfes; und
- Fig. 5: Teile eines Verfahrens gemäß einer Ausführungsform der vorliegenden Erfindung.

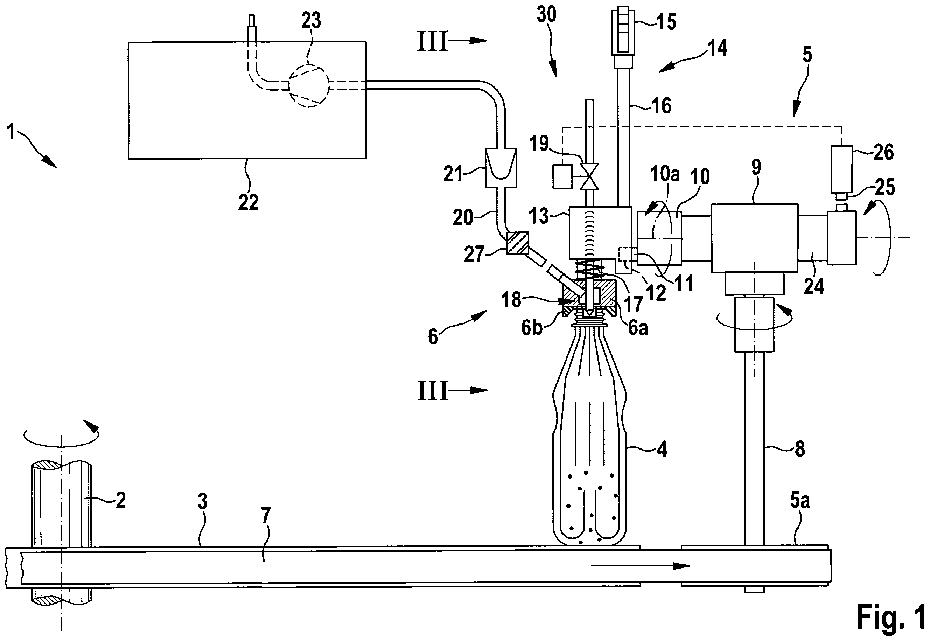

Fig. 1 zeigt in schematischer Form eine Seitenansicht einer Darstellung einer Vorrichtung die nicht als Teil der Erfindung betrachtet werden kann, da der Filter 21 nicht im Probenahmekopf angeordnet ist.

Die Vorrichtung 1 weist im dargestellten Ausführungsbeispiel der Fig. 1 einen durch eine Welle 2 angetriebenen Kreisförderer 3 für zu untersuchende Behälter 4, hier wiederbefüllbare PET-Flaschen (REFPET-Flaschen, Refilliable Polyethylenterephthalat-Flaschen), auf. In der Fig. 1 ist die Bewegung der Behälter 4 im Wesentlichen senkrecht zur Blattebene.

Mit dem Förderer 3 ist ein Getriebe 5 zur Betätigung einer Probenahmeeinrichtung 6 mit einem Probenahmekopf 6a verbunden, der zur Probenahme jeweils auf einen Behälter 4 aufgesetzt wird.

Im dargestellten Vorrichtung weist das Getriebe 5 zum Antrieb der Probenahmeeinrichtung 6 einen auf eine Getriebescheibe 5a wirkenden Antriebsgurt 7 auf. Die Getriebescheibe 5a ist mit einer Welle 8 verbunden, die auf ein nicht näher dargestelltes Winkelgetriebe 9 wirkt, mit dessen Ausgangswelle ein Excenter 10 verbunden ist, der mit einem exzentrisch zu seiner Drehachse 10a angeordneten Nocken 11 in eine Ausnehmung 12 (gestrichelt dargestellt) eines Halteteils 13 in Form eines Halteblocks für den Probenahmekopf 6a eingreift. Das Halteteil 13 ist über ein Führungsgestänge 14 gehalten und geführt.

Das Führungsgestänge weist einen Hebel 15 auf, der an einem Festpunkt 16 schwenkbar angelenkt ist. Mit seinem anderen Ende ist er gelenkig mit einer Führungsstange 16 verbunden, die fest und starr mit dem Halteteil 13 verbunden ist und diesen bei seiner durch den Nocken 11 verursachten Bewegung führt. Durch das Führungsgestänge wird der Umfang des Nockens 11 bei konstanter Winkelgeschwindigkeit gezeigt. Die ungleichmäßige horizontale Bewegung des Anlenkpunktes des Gestänges 14 am Halteteil 13 wird hinsichtlich des Probenahmekopfes 6a weitgehend kompensiert, so dass dieser der Bewegung der Behälter folgen kann.

Der Probenahmekopf 6a ist nachgiebig bzw. beweglich in Richtung der Führungsstange 16 am Halteteil 13 angeordnet, beispielsweise durch eine Schraubenfeder 17, wobei er gegenüber dem Haltekopf 13 auch eine gewisse Angularbeweglichkeit aufweisen kann.

In den Probenahmekopf 6a führt zunächst eine Druckluftlanze 18, deren Druckluftzufuhr über ein Ventil 19 steuerbar ist. Weiterhin mündet in den oberen Bereich des Probenahmekopfes 6a ein Probenahmeschlauch 20, der über einen Filter 21, der Fest- und Flüssigteilchen ausfiltert, mit einem Messsystem 22 verbunden ist. Nicht notwendigerweise, aber vorteilhaft, ist eine Saugpumpe 23 im Probenahmeschlauch-System vorgesehen.

Auf der dem Excenter 10 abgewandten Seite des Winkelgetriebes 9 findet sich eine weitere Abtriebswelle 24, die an ihrem Umfang eine Schaltnocke 25, welche über einen zugeordneten Impulsgeber 26 das Ventil 19 in der Druckluftlanze 18 steuert.

Die Stellung, in der sich das Halteteil 13 und damit der Probenahmekopf 6a in ihrer obersten Stelle befindet, wird als 0°-Stellung bezeichnet, während die Position, in der das Halteteil 13 und damit der Probenahmekopf 6a weitestmöglich nach unten gedrückt ist, als 180°-Stellung bezeichnet wird. In der 0°-Stellung ist die horizontale Zuordnung von Probenahmekopf 6a und Behälter in Bewegungsrichtung der Behälter derart, dass sich der Probenahmekopf etwa mittig zwischen zwei gegenüberliegenden Behältern befindet.

Die Bewegung des Behälters 4 sowie die hierzu parallel verlaufende Komponente (Horizontalkomponente) des Halteteils 13 und damit des Probenahmekopfes 6 sind miteinander synchronisiert, im dargestellten Ausführungsbeispiel durch das beschriebene Getriebe und Führungsgestänge, so dass beide Bewegungen in ihrer Geschwindigkeit und Richtung im Wesentlichen übereinstimmen.

Ein Behälter 4 gelangt mit seiner Öffnung unterhalb des Probenahmekopfes 6a in die 90°-Position desselben. Bei 105° umgreift der untere Rand des Probenahmekopfes 6a gerade den oberen Rand des Behälters 4. Bei etwa 120° setzt die Dichtung 6b des Probenahmekopfes auf dem oberen Rand des Probenahmekopfes 6a auf dem oberen Rand des Behälters auf. Zu dieser Bewegungsphase gelangt auch die Schaltnocke 25 zum Impulsgeber 26 und löst damit durch Öffnen des Ventils 19 einen Druckluftimpuls über die Düse der Druckluftlanze 18 in den Behälter aus, wodurch das in diesem befindliche Gas über die Probenahmeleitung 20 zum Messsystem 22 geleitet wird.

Bei weiteren Bewegungen bis 180° bewegt sich der Halteblock 13 weiter nach unten, während der Probenahmekopf 6a an einer weiteren Bewegung nach unten durch den oberen Rand des Behälters 4 gehindert wird. Durch die elastische Verbindung zwischen Probenahmekopf 6a und Halteteil 13 drückt letzterer den Probenahmekopf 6a fest gegen den oberen Rand des Behälters.

Bei 180° erreicht, wie gesagt, das Halteteil 13 seine niedrigste Position und übt damit die größte Andruckkraft auf den Probenahmekopf 6a aus.

Die Probe wird im Messsystem 22 analysiert, vorzugsweise spektroskopisch mittels UV- oder IR-Licht oder mittels Mikrowellenstrahlung, wobei allerdings auch andere Messmethoden, wie Massenspektrometrie oder die sogenannte TOF-Technik (Time of Flight) eingesetzt werden kann, wobei eine Ionisierung des aus dem Behälter 4 ausgetriebenen Gases und eine Beschleunigung der Gasmoleküle im elektrischen Feld erfolgt und die Gasarten sich aus den über eine vorgegebene Strecke ergebenden Flugzeiten bestimmt werden.

Mit der weiteren Bewegung über die 180°-Position hinaus hebt der Halteblock 13 sich wieder vom Behälter 4 ab und löst damit den Probenahmekopf 6a von der Behälteröffnung, wobei bei spätestens 270°der Probenahmekopf 6a die Oberseite des Behälters 4 völlig frei gibt, so dass der Behälter durch den Förderer 2 frei weitergefördert werden kann, während der Halteblock 13 und damit der Probenahmekopf 6 wieder in die 0°-Stellung zurückgeführt werden und nach erneutem Absenken in die 90°-Stellung wieder über die Öffnung des nächsten Behälters gelangen, so dass diesem in der beschriebenen Weise Gas zur Untersuchung entnommen werden kann.

Eine besonders bevorzugte Ausführungsform der Erfindung umfasst einen Probenahmekopf 6a mit einem Dichtkörper 6c, der so ausgeformt ist, dass seine an der Behälteröffnung anliegende, gewölbte Dichtfläche nach Innen und zur Behälteröffnung hin im Querschnitt konvex ausgebildet ist. Die Dichtfläche entspricht insbesondere derjenigen eines Kugelinnenseitenausschnitts. Hierdurch wird zuverlässig ein fluiddichtes Anliegen des Dichtkörpers 60 am Behälter 4 über einen weiten Winkelbereich gewährleistet. Dieser Dichtkörper 6c besteht aus einem nahezu verschleißfesten, gehärteten Material, wie Edelstahl, so dass er einem geringen Verschleiß unterliegt und so für geringe Störanfälligkeit sorgt. Vorteilhafterweise fährt die Druckluftlanze 18 vorteilhaft beim Aufsetzen des Probenahmekopfes 6a auf den Behälter 4 in den Behälter 4 ein, es federt also nur der abdichtende Probenahmekopf 6a nach, während die Druckluftlanze 18 exakt der Bewegung des Excenters 10 folgt.

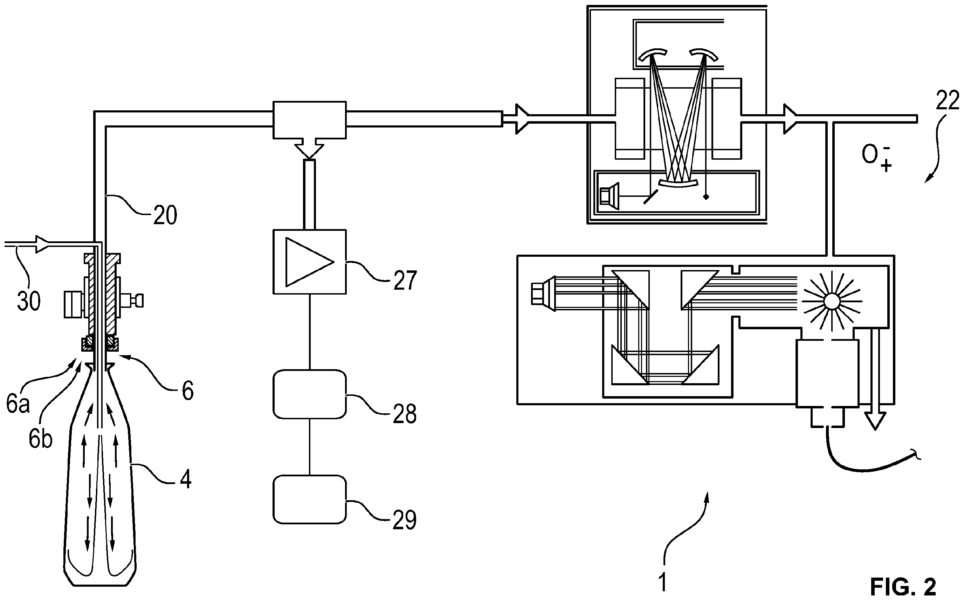

Fig. 2 zeigt einen prinzipiellen Aufbau einer Vorrichtung gemäß einer Ausführungsform der vorliegenden Erfindung.

In Fig. 2 ist eine Vorrichtung 1 im Wesentlichen gemäß Fig. 1 gezeigt. Über die Druckluftzuführleitung 30 wird Luft in den Behälter in Form einer Flasche 4 eingeblasen nachdem der Probenahmekopf 6 auf die Behälteröffnung aufgesetzt worden ist. Das durch das Aufsetzen bzw. Einblasen der Druckluft entweichende Gas in der Flasche 4 wird über eine Entnahmeleitung 20 einem Hochgeschwindigkeitsdrucksensor 27 zugeführt, der mit einer Datensammeleinrichtung 28 verbunden ist. Die Datensammeleinrichtung 28 ist mit einer Analyseeinrichtung 29 zur Analyse der erhaltenen Daten verbunden. Ggf. bereitet die Datensammeleinrichtung 28 die Rohdaten des Drucksensors 27 für die Analyseeinrichtung 29 auf, die ebenfalls auch mit dem Messsystem 22 verbunden sein kann. Darüber hinaus ist die Entnahmeleitung 20 mit dem Messsystem 22 verbunden, was beispielsweise das zweite Fluid mittels spektroskopischer Analyse untersucht. Die Analyseeinrichtung 29 und/oder die Datensammeleinrichtung 28 können dabei auch Teil des Messsystems 22 sein. Anhand des Resultats des Drucksensors 27 kann unterschieden werden, ob der Messvorgang zuverlässig durchgeführt wurde oder ob beispielsweise durch ein Leck Verfälschungen bei der Analyse des zweiten Fluids durch das Messsystem 22 möglich oder zu erwarten sind.

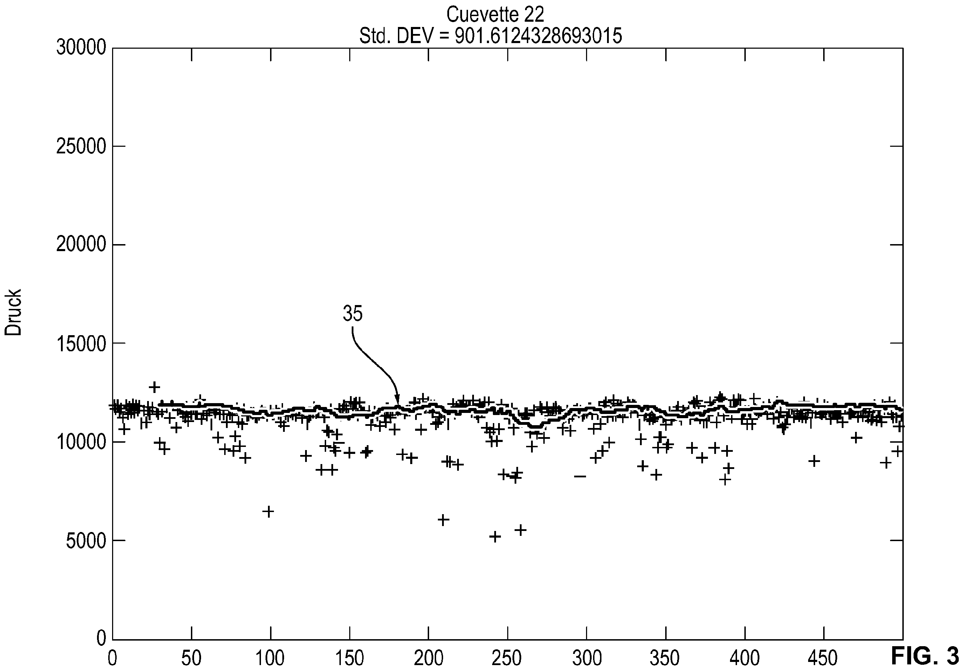

Fig. 3 zeigt Ergebnisse einer Druckmessung von 1,5 Liter REFPET-Flaschen bei einer Geschwindigkeit von 36.000 Flaschen pro Stunde während einer Zeitdauer von 30 Minuten.

In Fig. 3 ist eine Druckmessung von einer Vielzahl von 1,5 Liter REFPET-Flaschen gezeigt. Dabei werden durch die Vorrichtung 36.000 Flasche pro Stunde untersucht. Wie zu erkennen ist können defekte Flaschen, die möglicherweise das Messergebnis verfälschen, durch einen deutlichen Druckabfall vom Durchschnitt bzw. von der Hauptlinie 35 im Bereich von ca. 11.000 (beliebige Einheit) erkannt werden. Einzelne Messungen liegen bei weniger als der Hälfte des genannten durchschnittlichen Drucks. Die unterhalb der Hauptlinie 35 liegenden Druckmessungen korrespondieren zu einem für eine erfolgreiche Probenahme des zweiten Fluids zu niedrigen Druck. Die entsprechenden Behälter werden dann aussortiert, ggf. ein oder mehreren erneuten Messungen zugeführt, beispielsweise indem diese an den Eingang der Vorrichtung 1 gemäß Fig. 1 zurückgeführt werden bzw. endgültig nach erfolglosem wiederholtem Messen aussortiert werden.

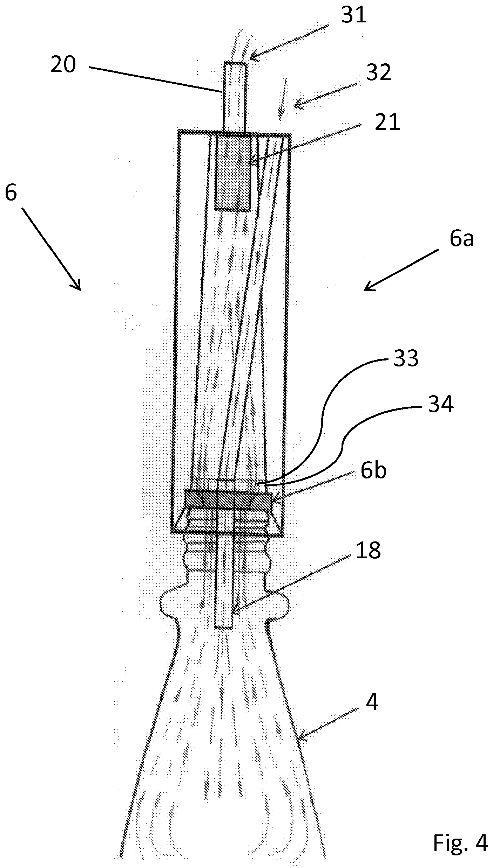

Fig. 4 zeigt in schematischer Form einen Ausschnitt einer weiteren Ausführungsform der vorliegenden Erfindung im Bereich des Probenahmekopfes.

In Fig. 4 ist im Wesentlichen ein Probenahmeeinrichtung 6 mit einem Probenahmekopf 6a im aufgesetzten Zustand auf eine PET-Flasche 4 gezeigt. Zur Dichtung des Übergangs zwischen Probenahmekopf 6a und PET-Flasche 4 gegenüber der Umgebung weist der Probenahmekopf 6a eine Dichtung 6b auf, sodass eine im Wesentlichen fluiddichte Verbindung zwischen Probenahmekopf 6a und PET-Flasche 4 ermöglicht wird. Diese Dichtung 6b ist in Form eines Dichtrings mit konischer Öffnung ausgebildet, wobei diese sich in Richtung auf die Öffnung der PET-Flasche 4 verjüngt.

Der Probenahmekopf 6a umfasst eine Fluidleitung zum Einleiten eines Fluids, hier in Form einer Druckluftlanze 18 zum Einblasen von, insbesondere ölfreier, Luft. Die Druckluftlanze reicht dabei vorzugsweise bis in den sich aufweitenden Bereich der PET-Flasche 4 zum Einblasen der Luft 32 hinein. Dir Druckluftlanze 18 wird dabei im Probenahmekopf 6a mittels eines Festlegungsrings 34 festgelegt, hier im Wesentlichen in der Mitte der der PET-Flasche 4 zugewandten Öffnung des Probenahmekopfes. Der Festlegungsring 34 weist in Umfangsrichtung Bohrungen 33 auf zum Durchtritt des zweiten Fluids, hier der verdrängten Luft 31. Die Druckluftlanze 18 ist dabei durch die Öffnung des Dichtringes 6b geführt, die radiale Anordnung der Bohrungen korrespondiert dabei zu der sich aufweitenden Öffnung des Dichtringes 6b, insbesondere derart, dass die vom Mittelpunkt des Dichtringes 6b bzw. Festlegungsringes 34 maximale radiale Erstreckung der Bohrungen 33 zu der maximalen Aufweitung des Dichtringes 6b, wie in Fig. 4 gezeigt, korrespondiert und die Bohrungen vollständig mit einem durch den Dichtring 6b durchtretenden Fluid beaufschlagt werden können.

Wird nun Luft 32 über die Druckluftlanze 18 in den Behälter 4 eingeblasen, entsteht ein Überdruck in dem Behälter 4 und die durch die eingeblasene Luft 32 verdrängte Luft 31 entweicht über die Öffnung des Behälters 4, tritt durch den Dichtring 6b in den Probenahmekopf 6a ein und passiert weiter einen Filter 21, der an der dem Behälter 4 abgewandten Seite des Probenahmekopfes 6a angeordnet ist, und tritt in eine Entnahmeleitung 20 ein, die mit einem Hochgeschwindigkeitsdrucksensor 27 zur Messung des Drucks der verdrängten Luft 31, und einem oder mehreren Messsystemen 22 verbunden ist.

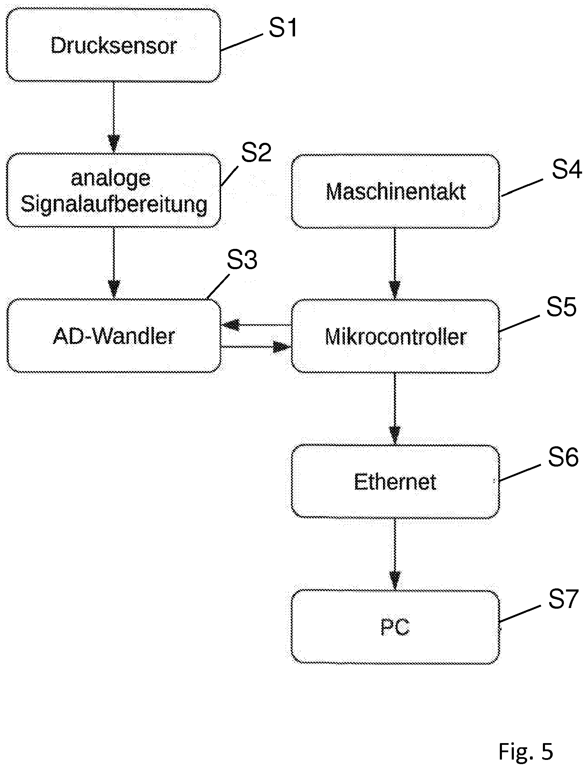

Fig. 5 zeigt Teile eines Verfahrens gemäß einer Ausführungsform der vorliegenden Erfindung.

In Fig. 5 sind im Wesentlichen die folgenden Schritte gezeigt: In einem ersten Schritt S1 misst ein Drucksensor den Druck des zweiten Fluids.

In einem zweiten Schritt S2 wird das vom Drucksensor ausgegebene Signal des Drucks analog aufbereitet, bspw. verstärkt, geglättet, oder dergleichen. Darüber können auch mehrere Signale summiert werden oder dergleichen.

In einem dritten Schritt S3 wird das aufbereitete Signal einem Analog-Digital-Wandler zugeführt und in ein digitales Signal umgewandelt.

In einem vierten Schritt S4 wird das digitale Signal von einem Mikrocontroller verarbeitet, der auch Daten, insbesondere die Taktrate einer Maschine, bspw. einer Förder- und Sortiereinrichtung für PET-Flaschen zur Zu-/und Abführung von Behältern zur Messung auf Inhaltstoffe in einem weiteren Schritt S5 erhält. Werden durch den Analog-Digital-Wandler weitere Daten, bspw. von anderen Messgrößen bereitgestellt, kann der Mikrocontroller mit dem Analog-Digital-Wandler kommunizieren und entsprechende Daten zur Übertragung an ihn auswählen. Der Mikrocontroller kann vor der Weiterleitung der digitalen Daten des Drucksensors diese mit dem Maschinentakt synchronisieren, sodass die Druckmessungen eindeutig einer bestimmten PET-Flasche zugeordnet werden können. So kann beispielsweise eine Verzögerung der Weiterleitung der digitalen Daten des Drucksensors erfolgen, wenn sich zwischenzeitlich der Arbeitstakt der Untersuchung der PET-Flaschen geändert hat. Der Mikrocontroller ist weiter bspw. über Ethernet mit einem Industrie-Personal Computer PC verbunden, der in einem weiteren Schritt S7 eine Auswertung des gemessenen Drucks und des Maschinentakts vornimmt. So kann bspw. bei Abweichungen des Drucks von einem vorgegebenen Wert anhand des Maschinentaktes die jeweilige PET-Flasche auch im weiteren Verlauf des Transport identifiziert werden und ggf. an den Eingang der Vorrichtung für eine erneute Messung rückgeführt werden oder auch direkt aussortiert werden. Der Industrie-PC kann bspw. eine Schwellwertüberprüfung durchführen, indem der vom Mikrocontroller bereitgestellte Messwert mit einem Schwellwert/Threshold verglichen wird und bei Unter- oder Überschreiten kann dann ein entsprechendes Signal automatisch ausgegeben werden, die Vorrichtung gestoppt werden oder dergleichen.

Zusammenfassend weist die vorliegende Erfindung den Vorteil auf, dass mittels des Drucksensors und einer Analyse des Drucks des entnommenen Fluids die Zuverlässigkeit bei der Erkennung von Fremdstoffen wesentlich erhöht wird.

### Bezugszeichenliste

- 1: Vorrichtung
- 2: Welle
- 3: Kreisförderer
- 4: Behälter
- 5: Getriebe
- 5a: Getriebescheibe
- 6: Probenahmeeinrichtung
- 6a: Probenahmekopf
- 6b: Dichtung
- 7: Impulsgeber
- 7: Antriebsgurt
- 8: Welle
- 9: Winkelgetriebe
- 10: Excenter
- 10a: Drehachse
- 11: Nocken
- 12: Ausnehmung
- 13: Halteteil
- 14: Führungsgestänge
- 15: Hebel
- 16: Führungsgestänge
- 16a: Festpunkt
- 17: Schraubenfeder
- 18: Druckluftlanze
- 19: Ventil
- 20: Probenahmeschlauch, Entnahmeleitung
- 21: Filter
- 22: Messsystem
- 23: Saugpumpe
- 24: Abtriebswelle
- 25: Schaltnocke
- 26: Impulsgeber
- 27: Hochgeschwindigkeitssensor
- 28: Datensammlung, Datenauswertung
- 29: Analyseeinrichtung
- 30: Druckluftzuführleitung
- 31: Luftfluss verdrängte Luft
- 32: Luftfluss eingeblasene Luft
- 33: Bohrung
- 34: Festlegungsring
- 35: Hauptlinie
- S1-S7: Verfahrensschritte

## Patentansprüche

1. Vorrichtung (1) zum Untersuchen von Behältern (4) auf Fremdstoffe, umfassend zumindest einen Probenahmekopf (6a), wobei der Probenahmekopf (6a) auf den zumindest einen Behälter (4) aufsetzbar ist und mittels des Probenahmekopfes (6a) eine Menge eines ersten Fluids (32) in den zumindest einen Behälter (4) einbringbar ist und wobei mittels des Probenahmekopfes (6a) eine Menge eines zweiten Fluids (31) zur Untersuchung auf Fremdstoffe aus dem zumindest einen Behälter (4) entnehmbar ist, wobei
zumindest ein Drucksensor (27) zur Überwachung des Drucks zumindest des zweiten Fluids (31) bei dessen Entnahme aus dem Behälter (4) stromabwärts des Probenahmekopfes (6a) angeordnet ist
und wobei ein Filter (21) zur Filterung des zweiten Fluids (31) im Probenahmekopf (6a) angeordnet ist, und wobei
der Probenahmekopf (6a) einen Dichtkörper (6b) aufweist zur Abdichtung einer Behälteröffnung während der Einbringung des ersten Fluids (32) und/oder während der Entnahme des zweiten Fluids (31), und
wobei eine Analyseeinrichtung (29, 22) zur Analyse des mittels des zumindest einen Drucksensors (27) gemessenen Drucks des zweiten Fluids (31) und zur Analyse des zweiten Fluids (31) in Bezug auf Fremdstoffe angeordnet ist.

2. Vorrichtung gemäß Anspruch 1, **dadurch gekennzeichnet, dass** der zumindest eine Drucksensor (27) fluidtechnisch stromabwärts des Probenahmekopfes (6a) und stromaufwärts der Analyseeinrichtung (29, 22) angeordnet ist.

3. Vorrichtung gemäß einem der Ansprüche 1-2, **dadurch gekennzeichnet, dass** die Analyseeinrichtung (29, 22) ausgebildet ist, mittels einer oder mehrere spektroskopischer Analysen des zweiten Fluids (31) Fremdstoffe zu ermitteln.

4. Vorrichtung gemäß einem der Ansprüche 1-3, **dadurch gekennzeichnet, dass** eine Fördereinrichtung (3) für die Behälter (4) angeordnet ist, derart, dass die Bewegung des Probenahmekopfes (6a) und die Bewegung der Behälter (4) miteinander synchronisierbar sind.

5. Vorrichtung gemäß einem der Ansprüche 1-4, **dadurch gekennzeichnet, dass** der Dichtkörper (6b) eine konische Bohrung aufweist, insbesondere wobei der Dichtkörper (6b) zumindest teilweise aus Gummi besteht.

6. Vorrichtung gemäß einem der Ansprüche 1-5, **dadurch gekennzeichnet, dass** eine Fluidbereitstellungseinrichtung (30) angeordnet ist, welche ausgebildet ist, das erste Fluid (32) in Form eines Gases oder Gasgemisches bereitzustellen, insbesondere wobei mittels der Fluidbereitstellungseinrichtung (30) das Gas in Form von Luft bereitstellbar ist.

7. Vorrichtung gemäß einem der Ansprüche 1-6, **dadurch gekennzeichnet, dass** der Drucksensor (27) als Hochgeschwindigkeitsdrucksensor ausgebildet ist, insbesondere wobei die Ansprechzeit des Drucksensors (27) weniger als 50 ms beträgt, insbesondere weniger als 25ms, vorzugsweise weniger als 10ms, besonders vorzugsweise weniger als 5ms, insbesondere zwischen 1 und 3ms.

8. Vorrichtung gemäß einem der Ansprüche 1-7, **dadurch gekennzeichnet, dass** das erste Fluid (32) mittels des Probenahmekopfes (6a) zentral in den zumindest einen Behälter (4) einbringbar ist.

9. Vorrichtung gemäß zumindest Anspruch 1, **dadurch gekennzeichnet, dass** eine Sortiereinrichtung angeordnet ist, die ausgebildet ist, anhand eines Resultats der Analyseeinrichtung (29, 22) Behälter (4) abweichend von einem vorab definierten Resultat auszusortieren, insbesondere wobei die Sortiereinrichtung ausgebildet ist, die Behälter (4) zumindest einmal erneut der Untersuchung auf Fremdstoffe zuzuführen.

10. Verfahren zum Untersuchen von Behältern (4) auf Fremdstoffe, umfassend die Schritte
a) Aufsetzen eines Probenahmekopfes (6a) auf eine Behälteröffnung zumindest eines Behälters (4),
b) Einbringen einer Menge eines ersten Fluids (32) in den zumindest einen Behälter (4) mittels des Probenahmekopfes (6a),
c) Entnehmen einer Menge eines zweiten Fluids zur Untersuchung auf Fremdstoffe mittels des Probenahmekopfes aus dem zumindest einen Behälter (4),
wobei der Probenahmekopf (6a) einen Dichtkörper (6b) aufweist zur Abdichtung einer Behälteröffnung während der Einbringung des ersten Fluids (32) und/oder während der Entnahme des zweiten Fluids (31), und wobei mittels zumindest eines stromabwärts des Probenahmekopfes angeordneten Drucksensors (27) zumindest der Druck des zweiten Fluids (31) bei der Entnahme aus dem Behälter (4) gemessen wird, und an eine Analyseeinrichtung (29, 22) zur Auswertung weitergeleitet wird und wobei das zweite Fluid (31) mittels eines Filters (21), der in Probenahmekopf (6a) angeordnet ist, gefiltert wird, insbesondere wobei Luft als erstes Fluid (32) bereitgestellt wird, insbesondere wobei Behälter (4) zumindest einmal erneut der Untersuchung auf Fremdstoffe zugeführt werden, wenn ein Resultat einer Analyseeinrichtung (29, 22) bei Analyse des zweiten Fluids (31) von einem vorab definierten Resultat abweicht.

11. Verfahren gemäß Anspruch 10, **dadurch gekennzeichnet, dass** bei der Entnahme des zweiten Fluids (31) der Druckverlauf des zweiten Fluids (31) während des Einbringens des ersten Fluids (31) gemessen wird, und/oder dass der Druck, insbesondere der Druckverlauf, des ersten Fluids (32) beim Einbringen in den zumindest einen Behälter (4) gemessen wird, insbesondere wobei die Druckverläufe sowohl des ersten Fluids (32) als auch des zweiten Fluids (31) gemessen und ausgewertet werden.

12. Verfahren gemäß einem der Ansprüche 10-11, **dadurch gekennzeichnet, dass** das zweite Fluid (31) optisch spektroskopisch untersucht wird und anhand der erhaltenen Daten der spektroskopischen Untersuchung mittels der Analyseeinrichtung (29, 22) die Fremdstoffe ermittelt werden.

13. Verfahren gemäß einem der Ansprüche 11-12, **dadurch gekennzeichnet, dass** der Druck des zweiten Fluids (31), insbesondere des Druckverlaufs des zweiten Fluids (31), mittels der Analyseeinrichtung (29, 22) anhand eines Soll-Ist-Vergleichs von Druckwerten, insbesondere von Druckverläufen, analysiert wird, wobei bei Unter- und/oder Überschreiten eines vorgegebenen Wertes die Untersuchung des Behälters (4) als fehlerhaft klassifiziert werden kann, insbesondere wobei der Soll-Wert des Soll-Ist-Vergleichs anhand einer Durchschnittsbildung einer Vielzahl von Druckmessungen festgelegt wird.

14. Verfahren gemäß Anspruch 13, **dadurch gekennzeichnet, dass** verschiedene Druckverläufe und/oder Durchschnittswerte von Druckmessungen zu unterschiedlichen Zeitpunkten analysiert werden.

## Claims

1. Device (1) for examining containers (4) for foreign substances, comprising at least one sampling head (6a), wherein the sampling head (6a) can be placed on the least one container (4) and by means of the sampling head (6a) a quantity of a first fluid (32) can be introduced into the at least one container (4) and wherein by means of the sampling head (6a) a quantity of a second fluid (31) for examination for foreign substances can be removed from the at least one container (4), wherein
at least one pressure sensor (27) for monitoring the pressure of at least the second fluid (31) when it is removed from the container (4) is arranged downstream of the sampling head (6a),
and wherein a filter (21) for filtering the second fluid (31) is arranged in the sampling head (6a), and wherein
the sampling head (6a) has a sealing member (6b) for sealing a container opening during the introduction of the first fluid (32) and/or during the removal of the second fluid (31), and
wherein an analysis device (29, 22) is arranged for analysing the pressure of the second fluid (31) measured by means of the at least one pressure sensor (27) and for analysing the second fluid (31) with regard to foreign substances.

2. Device according to claim 1, **characterised in that** the at least one pressure sensor (27) is arranged in technical fluid terms downstream of the sampling head (6a) and upstream of the analysis device (29, 22).

3. Device according to either claim 1 or claim 2, **characterised in that** the analysis device (29, 22) is constructed to establish foreign substances by means of one or more spectroscopic analyses of the second fluid (31).

4. Device according to any one of claims 1 to 3, **characterised in that** a conveyor device (3) for the containers (4) is arranged in such a manner that the movement of the sampling head (6a) and the movement of the containers (4) can be synchronised with each other.

5. Device according to any one of claims 1 to 4, **characterised in that** the sealing member (6b) has a conical hole, in particular wherein the sealing member (6b) at least partially comprises rubber.

6. Device according to any one of claims 1 to 5, **characterised in that** there is arranged a fluid provision device (30) which is constructed to provide the first fluid (32) in the form of a gas or gas mixture, in particular wherein the gas can be provided in the form of air by means of the fluid provision device (30).

7. Device according to any one of claims 1 to 6, **characterised in that** the pressure sensor (27) is constructed as a high-speed pressure sensor, in particular wherein the response time of the pressure sensor (27) is less than 50 ms, in particular less than 25 ms, preferably less than 10 ms, in a particularly preferred manner less than 5 ms, in particular between 1 and 3 ms.

8. Device according to any one of claims 1 to 7, **characterised in that** the first fluid (32) can be introduced by means of the sampling head (6a) centrally into the at least one container (4).

9. Device according to at least claim 1, **characterised in that** there is arranged a sorting device which is constructed, using a result of the analysis device (29, 22), to separate containers (4) which differ from a previously defined result, in particular wherein the sorting device is constructed to supply the containers (4) at least once again for the examination for foreign substances.

10. Method for examining containers (4) for foreign substances, comprising the steps of:
a) placing a sampling head (6a) on a container opening of at least one container (4),
b) introducing a quantity of a first fluid (32) into the at least one container (4) by means of the sampling head (6a),
c) removing a quantity of a second fluid for examination for foreign substances using the sampling head from the at least one container (4),
wherein the sampling head (6a) has a sealing member (6b) for sealing a container opening during the introduction of the first fluid (32) and/or during the removal of the second fluid (31), and wherein,
using at least one pressure sensor (27) which is arranged downstream of the sampling head, at least the pressure of the second fluid (31) during removal from the container (4) is measured and is transmitted to an analysis device (29, 22) for evaluation, and wherein the second fluid (31) is filtered using a filter (21) which is arranged in the sampling head (6a), in particular wherein air is provided as the first fluid (32), in particular wherein containers (4) are supplied at least once again for examination for foreign substances if a result of an analysis device (29, 22) differs from a previously defined result when the second fluid (31) is analysed.

11. Method according to claim 10, **characterised in that**, when the second fluid (31) is removed, the pressure path of the second fluid during the introduction of the first fluid (31) is measured, and/or **in that** the pressure, in particular the pressure path, of the first fluid (32) is measured during introduction into the at least one container (4), in particular wherein the pressure paths of both the first fluid (32) and the second fluid (31) are measured and evaluated.

12. Method according to any one of claims 10 to 11, **characterised in that** the second fluid (31) is examined in an optical spectroscopic manner and, with reference to the obtained data of the spectroscopic examination, the foreign substances are established by means of the analysis device (29, 22).

13. Method according to any one of claims 11 to 12, **characterised in that** the pressure of the second fluid (31), in particular the pressure path of the second fluid (31), is analysed by means of the analysis device (29, 22) with reference to a desired/actual comparison of pressure values, in particular of pressure paths, wherein, when a value falls below and/or exceeds a predetermined value, the examination of the container (4) can be classed as being defective, in particular wherein the desired value of the desired/actual comparison is determined with reference to an averaging of a large number of pressure measurements.

14. Method according to claim 13, **characterised in that** different pressure paths and/or average values of pressure measurements at different times are analysed.

## Revendications

1. Dispositif (1) pour déterminer la présence de substances étrangères dans des récipients (4), comprenant au moins une tête de prélèvement d'échantillon (6a), dans lequel la tête de prélèvement d'échantillon (6a) peut être posée sur l'au moins un récipient (4) et, au moyen de la tête de prélèvement d'échantillon (6a), une quantité d'un premier fluide (32) peut être introduite dans l'au moins un récipient (4), et dans lequel, au moyen de la tête de prélèvement d'échantillon (6a), une quantité d'un deuxième fluide (31) pour la détection de substances étrangères peut être prélevée dans l'au moins un récipient (4), dans lequel
au moins un capteur de pression (27), pour la surveillance de la pression au moins du deuxième fluide (31) lors de son prélèvement dans le récipient (4), est disposé en aval de la tête de prélèvement d'échantillon (6a)
et dans lequel un filtre (21), pour le filtrage du deuxième fluide (31), est disposé dans la tête de prélèvement d'échantillon (6a) et dans lequel
la tête de prélèvement d'échantillon (6a) comprend un corps d'étanchéité (6b) pour l'étanchéification d'une ouverture du récipient pendant l'introduction du premier fluide (32) et/ou pendant le prélèvement du deuxième fluide (31) et
dans lequel un dispositif d'analyse (29, 22) est prévu pour l'analyse de la pression du deuxième fluide (31), mesurée au moyen de l'au moins un capteur de pression (27) et pour l'analyse du deuxième fluide (31) afin de détecter des substances étrangères.

2. Dispositif selon la revendication 1, **caractérisé en ce que** l'au moins un capteur de pression (27) est disposé fluidiquement en aval de la tête de prélèvement d'échantillon (6a) et en amont du dispositif d'analyse (29, 22).

3. Dispositif selon l'une des revendications 1 - 2, **caractérisé en ce que** le dispositif d'analyse (29, 22) est conçu pour détecter les substances étrangères au moyen d'une ou de plusieurs analyses spectroscopiques du deuxième fluide (31).

4. Dispositif selon l'une des revendications 1 - 3, **caractérisé en ce qu'**un dispositif de convoyage (3) pour les récipients (4) est disposé de façon à ce que le déplacement de la tête de prélèvement d'échantillon (6a) et le déplacement des récipients (4) puissent être synchronisés entre eux.

5. Dispositif selon l'une des revendications 1 - 4, **caractérisé en ce que** le corps d'étanchéité (6b) présente un perçage conique, plus particulièrement dans lequel le corps d'étanchéité (6b) est constitué au moins partiellement de caoutchouc.

6. Dispositif selon l'une des revendications 1 - 5, **caractérisé en ce qu'**un dispositif de mise à disposition de fluide (30) est disposé, qui est conçu pour mettre à disposition le premier fluide (32) sous la forme d'un gaz ou d'un mélange de gaz, plus particulièrement dans lequel, au moyen du dispositif de mise à disposition de fluide (30), le gaz peut être mis à disposition sous la forme d'air.

7. Dispositif selon l'une des revendications 1 - 6, **caractérisé en ce que** le capteur de pression (27) est conçu comme un capteur de pression à haute vitesse, plus particulièrement dans lequel le temps de réponse du capteur de pression (27) est inférieure à 50 ms, plus particulièrement inférieur à 25 ms, de préférence inférieur à 10 ms, plus particulièrement de préférence inférieur à 5 ms, plus particulièrement entre 1 et 3 ms.

8. Dispositif selon l'une des revendications 1 - 7, **caractérisé en ce que** le premier fluide (32) peut être introduit au moyen de la tête de prélèvement d'échantillon (6a), de manière centrale dans l'au moins un récipient (4).

9. Dispositif selon au moins la revendication 1, **caractérisé en ce qu'**un dispositif de tri est disposé, qui est conçu pour trier, à l'aide d'un résultat du dispositif d'analyse (29, 22), les récipients (4) différemment d'un résultat préalablement défini, plus particulièrement dans lequel le dispositif de tri est conçu pour soumettre les récipients au moins à nouveau une fois à la détection de substances étrangères.

10. Procédé de détection de substances étrangères sur des récipients (4), comprenant les étapes suivantes
a) pose d'une tête de prélèvement d'échantillon (6a) sur une ouverture d'au moins un récipient (4),
b) introduction d'une quantité d'un premier fluide (32) dans l'au moins un récipient (4) au moyen de la tête de prélèvement d'échantillon (6a),
c) prélèvement d'une quantité d'un deuxième fluide pour la détection de substances étrangères au moyen de la tête de prélèvement d'échantillon dans l'au moins un récipient (4),
dans lequel la tête de prélèvement d'échantillon (6a) comprend un corps d'étanchéité (6b) pour l'étanchéification d'une ouverture du récipient pendant l'introduction du premier fluide (32) et/ou pendant le prélèvement du deuxième fluide (31) et dans lequel
au moyen d'au moins un capteur de pression (27) disposé en aval de la tête de prélèvement d'échantillon (6a), au moins la pression du deuxième fluide (31) est mesurée lors du prélèvement dans le récipient (4) et est transmise pour analyse à un dispositif d'analyse (29, 22) et dans lequel le deuxième fluide (31) est filtré au moyen d'un filtre (21), qui est disposé dans la tête de prélèvement d'échantillon (6a), plus particulièrement dans lequel de l'air est mis à disposition en tant que premier fluide (32), plus particulièrement dans lequel les récipients sont soumis au moins à nouveau une fois à la détection de substances étrangères lorsqu'un résultat d'un dispositif d'analyse (29, 22) s'écarte d'un résultat préalablement défini lors de l'analyse du deuxième fluide (31).

11. Procédé selon la revendication 10, **caractérisé en ce que**, lors du prélèvement du deuxième fluide (31), le tracé de la pression du deuxième fluide (31) est mesuré pendant l'introduction du premier fluide (31) et/ou **en ce que** la pression, plus particulièrement le tracé de la pression, du premier fluide (32) est mesuré lors de l'introduction dans l'au moins un récipient (4), plus particulièrement dans lequel les tracés de la pression aussi bien du premier fluide (32) que du deuxième fluide (31) sont mesurés et analysés.

12. Procédé selon l'une des revendications 10 - 11, **caractérisé en ce que** le deuxième fluide (31) est examiné de manière optique et spectroscopique et les substances étrangères sont déterminées à l'aide des données obtenues de l'examen spectroscopique au moyen du dispositif d'analyse (29, 22).

13. Procédé selon l'une des revendications 11 - 12, **caractérisé en ce que** la pression du deuxième fluide (31), plus particulièrement le tracé de la pression du deuxième fluide (31), est analysé au moyen du dispositif d'analyse (29, 22) à l'aide d'une comparaison consigne-réelle des valeurs de pression, plus particulièrement des tracés de pression, dans lequel, lors d'un passage en dessous et/ou d'un dépassement d'une valeur prédéterminée, l'examen du récipient (4) peut être classifié comme erroné, plus particulièrement dans lequel la valeur de consigne de la comparaison consigne-réelle est déterminée à l'aide d'une formation de moyenne d'une pluralité de mesures de pression.

14. Procédé selon la revendication 13, **caractérisé en ce que** différents tracés de pression et/ou valeurs moyennes de mesures de pression sont analysés à différents moments.
